Europäisches Patentamt

(19) European Patent Office

Office européen des brevets

(11) Publication number: **0 002 594**
**B2**

(12) **NEW EUROPEAN PATENT SPECIFICATION**

(45) Date of publication of the new patent specification: **15.02.89**

(51) Int. Cl.⁴: **A 61 K 7/16**

(21) Application number: **78300780.0**

(22) Date of filling: **08.12.78**

(54) Mouthwash.

(30) Priority: **12.12.77 US 859632**

(43) Date of publication of application:
**27.06.79 Bulletin 79/13**

(45) Publication of the grant of the patent:
**15.12.82 Bulletin 82/50**

(45) Mention of the opposition decision:
**15.02.89 Bulletin 89/7**

(84) Designated Contracting States:
**DE FR GB IT SE**

(56) References cited:
DE-A-2 255 177
DE-C-975 623
FR-A-2 185 388
FR-A-2 190 405
FR-A-2 258 865
FR-A-2 282 861
GB-A-1 288 819
GB-A-1 421 064

H. Janistyn's "Handbuch der Kosmetika und
Riechstoffe" I. Band: Die kosmetischen
Grundstoffe 3. Auflage page 30 u. 926
Fette, Seifen, Anstrichmittel, vol. 63 (7), pages 628-
630, 1961
Handbuch der Kosmetika und Riechstoffe, H.
Janistyn, III. Band: Die Körperpflegemittel, 2nd
Edition (1973), pages 782-794
Leaflet on Texapon Z, 1975, Henkel & Cie. GmbH
Remington's Pharmaceutical Sciences, 15th Edition

(73) Proprietor: **UNILEVER PLC, Unilever House
Blackfriars P.O. Box 68, London EC4P 4BQ (GB)**

(84) Designated Contracting States: **GB**

(73) Proprietor: **UNILEVER NV, Burgemeester
s'Jacobplein 1 P.O. Box 760, NL- 3000 DK
Rotterdam (NL)**

(84) Designated Contracting States: **DE FR IT SE**

(72) Inventor: **Elton, Craig Theodore, 1307 80th Street,
North Bergen Hudson New Jersey (US)**
Inventor: **Pader, Morton, 1358 Sussee Road,
Teaneck Bergen New Jersey (US)**

(74) Representative: **Doucy, Robert Henry, Unilever
PLC Patents Division P.O. Box 68 Unilever
House, London EC4P 4BQ (GB)**

(56) References cited: (continuation)
(1975), page 1245
Bailey's Industrial Oil and Fat Products, vol. 1, 4th
Edition, pages 313 + 315
Cosmetics Science and Technology, 2nd Edition,
vol. 1, pages 535 536
Harry's Cosmeticology, 7th Edition, pages 626-629

The file contains technical information submitted
after the application was filed and not included in
this specification

LIBER, STOCKHOLM 1989

**Description**

This invention relates to mouthwashes, and more particularly to that type of mouthwash which is effective against bacteria in the mouth.

Antibacterial mouthwashes have the primary purpose of reducing or removing bacteria that are usually present in large numbers in the oral cavity. Microbial populations in the mouth arise quite naturally, for example, from dental plaque, decaying food particles and from salivary stagnation. In many instances these microbes are responsible either directly or indirectly for malodour. Additionally, these microbes can present a source of infection and are believed by some investigators to be related at least in part to such problems as caries and periodontal disease.

A full treatment of mouthwash history and technology is presented by Rosenthal in Balsam et al <u>Cosmetics Science and Technology</u>, John Wiley and Sons, 2nd Edition, 1972, chapter 14.

It is well known that some of the malodourous elements of mouth aroma are generated by bacteria. It has been suggested that the germicidal agents commonly used in mouthwashes (quaternary ammonium compounds, phenolic compounds and sometimes favour components having germicidal action) exert their effect by destroying or inhibiting oral bacteria. However, some have proposed that the antibacterial agents can exert a deodorising effect on the oral cavity by a mechanism which is not related to their antibacterial activity.

Attempts to improve efficacy against oral malodour and other oral problems recently has resulted in increased stress on the importance of bacterial effectiveness. The compounds, however, that seem to demonstrate efficacy, at least at levels where bactericidal activity can be demonstrated, contribute significantly to favour, foam, clarity and irritancy problems. Lacking physiological acceptance, these products have proved unacceptable to many consumers for a number of reasons. Examples of antibacterial agents employed in mouthwashes include phenolic compounds such as beta-naphthol, thymol, carvacrol, chlorothymol, amy-, hexyl- heptyl- and octyl-phenols, hexyl resorcinol hexachlorophene and phenol; quaternary ammonium compounds such as quaternary morpholinium alkyl sulphates, cetyl pyridinium chloride, alkyl dimethyl benzyl ammonium chloride and alkyl trimethyl ammonium halides; and miscellaneous antibacterial components such as benzoic acid, formaldehyde, potassium chlorate, tyrothrycin, gramicidin, iodine and iodine-liberating compounds, and oxygen bearing compounds such as sodium perborate and urea peroxide. These compounds, however, have either a disagreeable taste, or are significantly effective only at levels where they cannot be effectively masked by flavourants. Many have been removed from the United States market by FDA action. Some are incompatible with other mouthwash ingredients, while still others have high and undesirable toxicity or sensitisation potential or are otherwise under suspicion of being unsafe for unsupervised human use.

Thus, presently available mouthwashes that are effective against bacteria suffer generally from the single major deficiency that they are at best physiologically unacceptable to many users and perhaps could present some potential hazard under certain conditions. At the very minimum a product must be acceptable to the user if it is to be used to full advantage.

It is known that some surface active agents, in particular the quaternary ammonium compounds, are toxic to bacteria based upon their ability to disrupt the permeability properties of the bacteria's cellular interface. Also included besides the quaternaries are certain anionic detergent actives and certain other miscellaneous antibacterial compounds. Among the anionic surface active agents that have been heretofore shown to demonstrate only a limited antibacterial activity is included sodium lauryl sulphate or more precisely sodium dodecyl sulphate. A discussion of these demonstrated activities is given in Lamanna et al <u>Basic Bacteriology</u>, 3rd Edition, Williams & Welkins Company, 1965, Baltimore, pages 921 - 925.

Various alkyl sulphates and in particular sodium lauryl sulphate have received wide use in various oral hygiene products, however, these agents have been employed infrequently in mouthwashes per se. When employed in mouth rinses they always have been employed for their foaming properties and when so with noted difficulty.

Scanlen et al US Patent No. 3 044 939, Menhart et al US Patent No. 3 250 686, McCane et al US Patent No. 3 488 419, Ergen US Patent No. 3 497 590, Januszewski et al US Patent No. 3 641 238, Prussin US Patent No. 3 954 962 and Howell US Patent No. 3 962 417 discuss various aspects of the use of sodium lauryl sulphate in dentifrice formulations.

Davies et al US Patent No. 3 087 857 disclosed a formulation containing 2 % sodium lauryl sulphate to clean and remove odours from the mouths of dogs. The sodium lauryl sulphate is present to provide a cleaning effect as deodorancy and antibacterial action is provided by the inclusion of preservatives and bacteriocidal agents such as soluble penicillin and neomycin.

Moeller et al US Patent No. 3 644 613 discloses mouthwashes containing 0.1 to 2 % of a sudsing agent; however, it is apparent that the sole function of the sudsing agent is to supply foam and that the character of the sudsing agent is not critical.

Lavinsky et al US Patent No. 3 462 525 discloses the use of olefin sulphonates as a replacement for sodium lauryl sulphates which are objectionable due to well noted "orange juice effects".

It is an object of the instant invention to provide an antibacterial mouthwash that is physiologically acceptable to the user and which does not rely upon conventional germicige agents to provide antibacterial action. All percentages herein are by weight unless specified otherwise.

According to the present invention there is provided a physiologically acceptable, germicide-free, water-

2

based clear mouthwash comprising:

(a) 0.1 to 0.5 % of an essential oil favour consisting of spearmint or peppermint or a mixture thereof;

(b) 0.1 to 0.6 % of an alkyl sulphate anionic surfactant mixture wherein said mixture consists essentially of a dodecyl sulphate salt and a tetradecyl sulphate salt wherein the cationic moiety of said salts is sodium, potassium, magnesium, ammonium or substituted ammonium ions, and said dodecyl sulphate is present in said mixture in a weight ratio to said tetradecyl sulphate of 75 : 1 to 1 : 1; and

(c) 5 to 20 % of ethanol.

The composition as set forth above provides for a highly acceptable pleasant tasting mouth rinse that at the same time affords a high degree of antibacterial action. Additionally, this unique and specific combination affords several unique benefits heretofore unexpected that will become apparent from the following description.

By the term germicide-free, it is meant that the mouthwash of the instant invention is free from the known and conventional germicides previously associated with mouthwashes. As stated previously, mouthwashes having antibacterial activity generally contain chemical compounds known to be active against a wide spectrum of microorganisms. With the exception of those rinses which rely on certain favour components such as oil of cinnamon, cassia, clove, eucalyptus, thyme, peppermint, anise, and wintergreen as well as the derivatives of these oils such as methanol, thymol, eucalyptol, anethole and methyl salicylate, to provide some minimal antibacterial effects, these rinses generally contain one or more compounds selected from categories of useful phenol compounds and quaternary ammonium compounds. The useful phenol compounds include (but are not limited to) for example beta-naphthol, thymol, carvacrol, chlorothymol, amy-, hexyl-, heptyl- and octylphenols, hexyl resorcinol, hexachlorophene and phenol itself. Examples of commercially important quaternary compounds include cetyl pyridinium chloride and alkyl dimethyl benzyl ammonium chloride. Additional known antibacterial agents not under these general headings include for example benzoic acid, formaldehyde, potassium chlorate, tyrothrycin, gramicidin, iodine and iodine-liberating compounds, boric acid, chlorine-liberating compounds, 8-hydroxyquinoline, nitrofurazine, organic mercury compounds, sodium perborate, urea peroxide and other oxygen-liberating compounds. Thus, with the possible exception of favour ingredients which will not be present at a level sufficient to contribute any significant antibacterial action, the mouthwash of the present invention will be free of known antibacterial agents as exemplified above.

By the term physiologically acceptable, Applicants mean that the mouthwash under conditions of intended use is safe and organoleptically tolerable in the oral cavity, having no significant side effects either orally or systemically when used as directed.

Applicants have found that antibacterial mouthwashes, free of essentially all bitter flavour and containing those essential flavour oils which are the most associated with freshness by the general public can be prepared by employing various lauryl sulphate salts selected from sodium, potassium, magnesium, ammonium and substituted ammonium lauryl sulphate and mixtures thereof in place of the customarily employed germicides discussed above. Applicants have further found that in addition to producing an antibacterial mouthwash, the resulting product is physiologically acceptable and in fact is readily accepted by the user.

The mouthwash contains a spearrnint or peppermint flavouring oil or mixture thereof. These flavours are well liked by the consuming public as representing and furnishing a fresh mouth feel. They comprise essential oils (namely spearmint and/or peppermit) which provide excellent persistent favour notes effective for residual odour masking. It is important to note that this type of flavour system has heretofore had only limited and generally unsuccessful use in antibacterial mouthwashes since such flavours were heretofore believed to be incompatible with known germicides and more importantly were believed to be extremely difficult to solubilise at least if essential oils were being employed. Additionally when solubilised, if at all, such solubilisation was achieved by the use of high levels of nonionic emulsifier which provided no added efficacious benefit to the product and in many instances contributed to off flavor.

This point itself is worthy of further amplification as the discovery of the ability to use such essential oil flavour mixtures in an aqueous base without the use of a nonionic emulsifier or nonionic surfactant to keep them in solution is quite contrary to general beliefs held within the art. Even more significant is the fact that this solubility is achieved at relatively low levels of anionic surfactant.

Heretofore it was believed that flavour mixtures of this kind could only be held in solution by the use of exceedingly high levels of surfactants, levels of at least 2 %, or more, which levels when employing the anionic surfactants and in particular alkyl sulphates were known to be potentially harmful in a product of this kind. While levels of sodium lauryl sulphate in this higher range have been employed in toothpaste products, use of such levels have been unacceptable in mouthwashes for safety reasons. Thus for all practical purposes the stabilisation of essential mint oils in an aqueous base by using small amounts (i.e. about 0.1 to less than 1 %) of a lauryl sulphate was heretofore believed impossible.

Applicants surprisingly have found that substantial amounts of such flavours, i.e. 0.1 to preferably 0.2 to 0.5 % by weight of a substantially aqueous solution can be solubilised by the inclusion in said solution of 0.1 to 0.6 %. by weight of said mouthwash, of an alkyl sulphate anionic surfactant mixture wherein said mixture consists essentially of a dodecyl sulphate salt and a tetradecyl sulphate salt wherein the cationic moiety of said salts is selected from sodium, potassium, magnesium, ammonium, and substituted ammonium ions and mixtures thereof, and wherein said dodecyl sulphate is present in said mixture in a weight ratio to said tetradecyl sulphate of 75 : 1 to 1 : 1. By a substantially aqueous solution is meant the solution contains 45 to 95 % water

3

and contains no more than 20 % ethanol. Thus, Applicants surprisingly have found that with their system of using a dodecyl sulphate is conjunction with a tetradecyl sulphate as a replacement for known germicides, they are now able to solubilise spearmint and peppermint flavours as well as provide antibacterial activity. Additionally, and more surprisingly, this end has been achieved with remarkably low levels of these sulphates and without the need for additional nonionic emulsifiers.

It has been found that the flavour mixture may be present in an amount of 0.1 to 0.5 % of the mouthwash. Preferably, the level of the favour should be 0.2 to 0.5 %, with the most desirable level being about 0.25 %.

The flavourant is characterised by containing major portions of menthol, methyl salicylate, oil of peppermint, and spearmint oil both natural and synthetic along with various flavour modifiers which the maker may wish to employ to give his product a distinctive note. By a major portion is meant at least about 50 % of the favour mixture should be made up by at least one or a mixture of these ingredients.

The amount of alkyl sulphate used is 0.1 to 0.6 %. Levels of alkyl sulphate less than the level of about 0.1 % do not demonstrate adequate antibacterial activity or solubilising effect of the flavouring oils, and levels much higher than about 0.6 % may lack physiological acceptability. While a level of the alkyl sulphate mixture of between 0.1 to 0.6 % is adequate, it is preferred to have the sulphate mixture present at a level of about 0.2 to about 0.4 % with the most desirable level being about 0.3 %.

While sodium dodecyl sulphate and sodium tetradecyl sulphate are the preferred salt forms the cationic moiety of the respective salts may also be potassium, magnesium, ammonium or substituted ammonium ions or mixtures thereof.

While it has been known that sodium dodecyl sulphate will exhibit some antibacterial activity, use of such an active for the replacement of high active germicides in a mouthwash was heretofore uncontemplated in the art. Generally, it was believed that levels associated with efficient killing action would be intolerable to the user, i.e. levels of more than 1 %. Applicants have found that levels of lauryl sulphates approaching 1 % are indeed too high to be tolerable in a mouthwash product. Applicants, however, have discovered that more than adequate antibacterial action can be achieved at levels much lower than heretofore believed as measured against the aforestated criteria.

The function of the ethanol is to add bite and refreshment to the mouthwash and may in some instances, in a limited way, act to enhance the solubilisation of certain flavour oils. Secondly, the alcohol enhances the cleansing efficacy. The mouthwash contains 5 to 20 % by weight of ethanol. Preferably, however, the rinse should contain 10 to 15 %. More desirably, the rinse should contain 12 to about 13 % of the alcohol. All levels of alcohol are expressed as percent 190 proof alcohol contained in the mixture.

Where the product may be subject to low temperature storage it is desirable that the dodecyl sulphate should be present in a weight ratio to the tetradecyl sulphate of act 4 : 1 to 1 : 1. Using such more limited ratios it is ensured that the product will remain water clear after storage at 2° C for a period of at least 7 days.

Nonionic emulsifiers may be included to modify flavour effect and initial taste. These ingredients are customarily added to conventional mouthwashes to solubilise flavour. Due to the nature of the present invention, they are not required for this purpose. However, they have been found in the present invention to provide some degree of flavour modification effect. Generally, these emulsifiers may be employed at a level of from 0 to 3 % by weight of the mouthwash; however, it is preferable to employ at least 0.1 %. While the exact emulsifer is not critical, one should be cautioned that not all nonionic emulsifiers will work, and in fact some will seriously detract from stability. Known to provide adequate results are such nonionic emulsifiers such as polyoxyethylene 20 sorbitan monolaurate, polyoxyethylene 20 stearyl ether, polyoxyethylene 20 isohexadecyl ether, polyoxyethylene 100 stearate, polyoxyethylene 10 stearyl ether and mixtures thereof. Polyoxyethylene 20 sorbitan monolaurate is the preferred nonionic emulsifier. While not all of the commercially available emulsifiers will work, these compounds are very familiar to one skilled in the art and it is a simple task for one skilled in the art to determine those which will be compatible. While the aforementioned disclosure of various emulsifiers will work, Applicants in no way wish to suggest that their invention is limited to just these emulsifiers as any nonionc emulsifier that will provide a benefit and that is compatible with the whole formulation is within the scope of the invention.

Humectants may be incorporated at levels of from 0 to 25 % by weight of the mouthwash. The function of the humectant is primarily to add "body" or mouthfeel to the mouthwash thereby making it more pleasant to use. As with the nonionic emulsifiers these compounds are readily familiar to those skilled in the art and many variations of those ingredients can successfully be employed by one skilled in the art with simple experimentation. Preferably, the humectant is present at a level of at least act 5 %, such as 5 to 20 %. Desirably, the humectant should be present at a level of 10 to 15 % with the most desirable level being about 12 %. The preferred humectant is sorbitol. Additional humectants that may be used include but are not limited to glycerol, propylene glycol and corn syrup.

An alkali metal halide, preferably sodium chloride may be present from 0 to 2 % by weight of the mouthwash. The halide salt serves as an electrolyte and contributes to the overall flavour. Preferably, this salt should be present at a level of 0.01 to 0.1 %.

Additionally, the mouthwash may contain a buffering salt pair to control the pH of the final product. The composition of the salt pair is highly variable and one skilled in the art can with simple experimentation arrive at various salt pairs that will be functional and that will not detract from the overall compositions. Generally, the salt pair should be capable of buffering the mouthwash to a pH of between 3 and 8 and should be present in said mouthwash at a level of 0 to 2 %. Preferably, the buffering salt pair should be present at a level of 0.01.

to 0.1 %. The preferred salt pair is a mixture of sodium acetate and acetic acid. When employing this mixture, the ratio of the sodium acetate to the acetic acid can vary widely so long as the resultant ratio is capable of producing a pH within the desired range. Preferably, however, the weight ratio of sodium acetate to acetic acid is about 3 : 2.

Artificial sweeteners, as well as natural sweeteners, may be employed to round out the resulting product as many users prefer a sweeter product than one containing just essential oil flavours. While sodium saccharin is the preferred sweetening agent, any compatible sweetening agent is contemplated within the scope of the invention. When employing sodium saccharin 0 to 2 % by weight of the mouthwash may be employed. In employing any other compatible sweetening agent, any amount equivalent to producing an equivalent level of sweetening to the 0 to 2 % sodium saccharin will suffice.

The water of the mouthwash serves as a fluid base and functions as a flushing medium to wash away food particles cleansed from the mouth by the mouthwash. The level of water content of the rinse will generally range from 94.8 % to 45 % by weight of the product, preferably 85 % to 45 %.

While the manner of mixing the ingredients of mouthwash is not critical, it is preferred to prepare alcohol and water phase premixes and to then mix these phases. More preferably, the alcohol premix should contain only a portion of the alcohol of the product with the remaining alcohol of the product being added subsequent to the comixture of the two premixes. Filtration may be employed at this point to enhance the clarity of the resulting product. Generally, the alcohol phase should comprise at least a portion of the alcohol of the final product, the favour mixture and the nonionic emulsifier if an emusifier is employed. The water phase should contain the water and the alkyl sulphate mixture and, if employed, sweetening agent, alkali metal halide salt, buffering salt pair, humectant and colourant if desired.

The mouthwash of the invention is useful in reducing malodour in the mouth associated with the ingestion of foods containing onion and garlic, an effect obtained with a relatively low level of alcohol and without bitter or astringent substances and an effect heretofore not demonstrated by conventional mouthwashes.

The following examples illustrate the invention. All parts are by weight unless specified otherwise.

**Example 1**

A mouthwash comprising a water base containing 12.5 % ethanol, 0.25 % of an essential oil flavour mixture comprising a mixture of predominantly spearmint and peppermint essential oils and 0.3 % of sodium dodecyl sulphate was prepared by first dissolving the flavour oils in the alcohol and second dissolving the sodium lauryl sulphate and thirdly comixing the alcohol and water premixes. The result was a turbid suspension at room temperature that would not clear. A GLC analysis of this sodium dodecyl sulphate indicated the following composition of alkyl sulphate homologues:

|  | % |
| --- | --- |
| Sodium dodecyl sulphate | 99.7 |
| Sodium tetradecyl sulphate | 0 |
| Miscellaneous impurities | 0.3 |

Thus the sodium dodecyl sulphate did not solubilise the essential oil flavour mixture.

A mixture according to the above was then compounded in the same manner but substituting for the sodium dodecyl sulphate a sodium dodecyl sulphate-sodium tetradecyl sulphate mixture having the composition:

|  | % |
| --- | --- |
| Sodium dodecyl sulphate | 97.5 |
| Sodiumtetradecylsulphate | 1.3 |
| Miscellaneous $C_{10}$, $C_{16}$ and $C_{18}$ sulphates | 1.2 |

This mixture produced a water clear solution. The solution was stable at room temperature but became less clear under conditions of reduced temperature (2°C) and extended storage (7 days).

**Example 2**

Mixtures according to Example 1 were prepared using alkyl sulphate mixtures as indicated:

|  | Example | | |
|--|--|--|--|
|  | 2A | 2B | 2C |
| Sodium decyl sulphate | Trace | Trace | Trace |
| Sodium dodecylsulphate | 65.5 % | 71.5 % | 66.9 % |
| Sodium tetradecyl sulphate | 27.1 % | 28.2 % | 32.9 % |
| Sodium hexadecyl sulphate | 7.3 % | Trace | Trace |

The resulting mouthwashes were water clear and stable at room temperature and remaining so after 7 days at 2°C (35°F).

**Example 3**

14 solutions coded 3A to 3N were made up as indicated in Table I.

**Table I**

| Solution | % dodecyl sulphate-tetradecyl sulphate mixture having a $C_{12} : C_{14}$ ratio between about 4 : 1 to 1 : 1 | % doublemint essential oil favour mixture* | % Ethanol |
|--|--|--|--|
| 3A | 0 | 0 | 12.5 |
| 3B | 0 | 0.25 | 12.5 |
| 3C | 0.08 | 0 | 0 |
| 3D | 0.08 | 0 | 12.5 |
| 3E | 0.08 | 0.25 | 0 |
| 3F | 0.08 | 0.25 | 12.5 |
| 3G | 0.3 | 0 | 0 |
| 3H | 0.3 | 0 | 12.5 |
| 3I | 0.3 | 0.25 | 0 |
| 3J | 0.3 | 0.25 | 12.5 |
| 3K | 0.6 | 0 | 0 |
| 3L | 0.6 | 0 | 12.5 |
| 3M | 0.6 | 0.25 | 0 |
| 3N | 0.6 | 0.25 | 12.5 |

* A mixture containing a mixture of spearmint and peppermint essential oils.

The resulting solutions were evaluated for bacterial activity under the Bactericidal Contact Time (BCT) test method as follows:

1. 1 ml of stock (A) were placed in a medication tube (25 x 100 mm) in a rack held at the desired temperature.
2. 1 ml of culture preparation (B) were added and timing started using a stop watch. For multiple testing, mixtures can be inoculated at 15-second intervals and sampled accordingly.
3. Breed calibrated loops (Will Corporation) were used for withdrawing samples at predetermined intervals. If, by design, the initial count was $10^7$/ml, a 0.001 ml loop was used; if $10^6$/ml, a 0.01 ml loop was used. Several loops were used in rotation to allow time to cool after flaming.
4. Each loop sample was immediately placed into 5 ml molten agar in oval tubes (special order, Bellco Glass Incorporated), held at about 48°C in a water bath. The use of Morton closures allows for ease in manipulation of tubes during performance of the test. Racks are obtainable from George H. Wahmann Manufacturing Company, Baltimore.
5. The medium employed was Microinoculum Broth + 1.5 % agar. The clarity of this medium is exceptional, its richness fosters the development of large discrete colonies, and no additional neutralisers are apparently necessary.
6. The agar containing the sample was gently mixed and the tube slanted in a rack to allow hardening. The rack of tubes was then inverted and incubated at 37°C for colony development. Counts were made in the same manner as standard plate counts. employing an oval tube adapter (1957) for the Quebec Colony Counter for ease in counting.
7. Initial density of organisms was determined by diluting culture preparations (B) $10^3$. 1 ml of this dilution was added to 1 ml of sterile 0.1 % peptone water (instead of test solution) in a control medication tube and sampled in replicates (at least 3) by appropriate loop. The average colony count obtained represents the 99.9 % end-point in any of the test mixtures. Table II illustrates the mathematics involved to arrive at this end-point. If of interest, a count of one-tenth that in the control would be the 99.99 % end-point.

6

8. To equate the BCT Test with the Chambers Test (1956, 1965), the initial count should be $10^8$/ml and a 0.01 ml loop would be employed to an end-point of 99.99 % kill.

9. Results are recorded as a function of time to effect a certain degree of kill, rather than as % reduction values after a set time interval.

**Table II**

30 × $10^6$/ml at start                                          3 × $10^6$/ml at start

99.9% kill          $10^{-3}$ dilution              99.9% kill          $10^{-3}$ dilution
                    (control)                                           (control)

30,000/ml                                          3,000/ml

0.001 ml loop                                      0.01 ml loop

30 colonies in oval tube

Additionally, all the test solutions were evaluated for physioligical acceptability. Results of these evaluations are included in Table III.

**Table III**

| Solution | BCT Time to 99.9 % kill | Physiological* Acceptability |
|---|---|---|
| 3A | > 5 min | A |
| 3B | > 5 min | UA |
| 3C | > 5 min | UA |
| 3D | > 5 min | UA |
| 3E | > 5 min | UA |
| 3F | > 5 min | UA |
| 3G | 15 sec | UA |
| 3H | 15 sec | UA |
| 3I | 15 sec | UA |
| 3J | 15 sec | A |
| 3K | 15 sec | UA |
| 3L | 15 sec | UA |
| 3M | 15 sec | UA |
| 3N | 15 sec | A |

A = acceptable; UA = unacceptable.

Less than 0.1 % of the alkyl sulphate mixture could not stabilize the three-component mixture (3F) and none of the solutions showed an adequate kill time under the BCT Test.

At the 0.3 % level of alkyl sulphate mixture, all solutions were stable and produced excellent kill times under the BCT Test. 3G was unacceptable as it was an unflavoured detergent solution as was 3H. 3I was unacceptable as it lacked alcohol to provide bite.

7

At the 0.6 % level of alkyl sulphate mixtures, all solutions were stable and produced excellent kill times under the BCT Test. Solutions 3K, 3L and 3M were unacceptable for reasons similar to the corresponding 3G, 3H and 3J.

The clarity of solutions 3G to 3N were not affected by storage at 2°C for 7 days.

## Example 4

2 mouthwash compositions, 4A and 4B were made up as follows:

| Ingredient | 4A % | 4B % |
|---|---|---|
| Ethanol, 190 proof | 12.5000 | 12.5000 |
| Doublemint essential oil favour mixture | 0.2500 | 0.2500 |
| Polyoxyethylene 20 sorbitan monolaurate | 0.2500 | - |
| Sodium alkyl sulphate mixture ($C_{12} : C_{14}$ ratio about 2.5 : 1) | 0.3000 | 0.3000 |
| Sorbitol | 12.0000 | 12.0000 |
| Sodium saccharin | 0.0650 | 0.0650 |
| Sodium chloride | 0.0500 | 0.0500 |
| Sodium acetate | 0.0300 | 0.0300 |
| Acetic acid | 0.0200 | 0.0200 |
| Colourant | 0.00085 | 0.00085 |
| Water | ← to | 100 → |

As can be seen, the formulations are identical with the exception that formula 4B does not contain a nonionic emulsifier.

The products were panel tested using 30 panellists divided randomly into 2 groups of 15. Product 4B was judged to produce the favour effect more quickly and product 4A was perceived as being more burning to the mouth.

In a panel test for overall acceptability both mouthwashes were equally and generally well received, inclusion of the nonionic emulsifier in product 4A being judged to help round out the overall favour perception.

## Example 5

The mouthwash of Example 4A was compared to certain commercially available mouthwashes containing various known germicides using the buccal tissue count method described below.

### Buccal Tissue Count Method

Approximately 12 subjects per product (13 were used on a control which was the mouthwash ot Example 4A without alkyl sulphate) were recruited and instructed not to brush their teeth on the morning of the test, and to report to the test area as soon as they arrived on that morning. The test was then conducted as follows:

General procedure
1. A curette sample of buccal tissue was first taken from each panellist, and transferred to 10 ml sterile 0.1 % peptone water ($10^{-1}$ dilution of the sample) solution.
2. The subject then rinsed with 15 ml of the mouthwash for 30 seconds, followed by 2 brief water rinses, and a second sample of buccal tissue was taken immediately thereafter. The time of day was recorded on a card and a schedule prepared for return visits for each subject. This completed the product usage part of the test, and the panellists returned according to interval-sampling schedules that they were given. At the times designated, they returned to the test area for additonal tissue sampling. All sample tubes were refrigerated between intervals.
3. Since bacterial counts returned to baseline within 2 hours, unless the product used contained an antibacterial agent, the schedule of interval-sampling was normally 2, 3 and 4 hours beyond zero- time for differentiation of test products. When testing non-antibacterial products, however, sampling intervals were narrowed to include a 1 hour, or even a 2 hour post-usage sample.
4. The accumulated samples were then plated in Brain Heart Infusion Agar (or any other rich medium of choice) at dilutions appropriate to the product and interval represented. For antibacterial products, countable plates were normally obtained by the following dilution scheme:

| | | |
|---|---|---|
| Before | $10^{-3}$ and $10^{-4}$ | 0.1 ml (of $10^{-1}$ sample dilution) in 10 ml = $10^{-3}$; 1 and 0.1 ml plated |
| After | $10^{-1}$ and $10^{-2}$ | 1 and 0.1 ml plated directly |
| 2 hours | $10^{-2}$ and $10^{-3}$ | 1 ml in 10 ml = $10^{-2}$; 1 and 0.1 ml plated |
| 3 hours | $10^{-3}$ and $10^{-4}$ | as the "before" sample |
| 4 hours | $10^{-3}$ and $10^{-4}$ | |

For non-antibacterial products, an appropriate dilution scheme was:

| | | |
|---|---|---|
| Before | $10^{-3}$ and $10^{-4}$ | |
| After | $10^{-1}$ and $10^{-2}$ | |
| 1/2 hour | $10^{-2}$ and $10^{-3}$ | obtained in same manner as above |
| 1 hour | $10^{-2}$ and $10^{-3}$ | |
| 2 hours | $10^{-3}$ and $10^{-4}$ | |
| 3 hours | $10^{-3}$ and $10^{-4}$ | |

5. The finished plates were allowed to harden, inverted, and incubated (at 32°C) for 7 days. Colonies were counted, converted to count $\times 10^3$/BT sample/subject, with the data computer. The analyses include group geometric means, % change from baseline per interval, P value and 95 % confidence limits.

6. As a general rule, antibacterial products maintain a reduced count ($P \geqslant 0.95$) for at least 2 hours and are differentiated on the basis of how long the effect lasts. The effect of non-antibacterial products is normally lost within 2 hours.

The buccal time count results are shown in Table IV.

**Table IV**

| Product | Active | Criteria | 0 | Hours After Single Use | | |
|---|---|---|---|---|---|---|
| | | | | 0.5 | 1 | 2 |
| Water | none | % reduction | 37.7 | | | |
| | | P value | 0.98 | | | |
| Control | none | % reduction | | 67.3 | 43.8 | -41.6 |
| | | P value | | 0.99 | 0.80 | 0.80 |
| 4A | 0.3 % alkyl sulphate mixture | % reduction | 97.1 | 95.3 | 90.9 | 68.4 |
| | | P value | 0.99 | 0.99 | 0.99 | 0.99 |
| L* | zinc chloride | % reduction | 79.3 | 74.6 | 67.9 | -8.5 |
| | | P value | 0.99 | 0.99 | 0.99 | <0.75 |
| M* | cetyl pyridinium chloride | % reduction | 99.9 | | | 86.9 |
| | | P value | 0.99 | | | 0.99 |
| S* | cetyl pyridinium chloride and domiphene bromide | % reduction | 99.9 | | 97.3 | 95.3 |
| | | P value | 0.99 | | 0.99 | 0.99 |

As can be seen, the mouthwash of the present invention (product 4A) provided equivalent antibacterial activity to commercial products containing various known actives including cetyl pyridinium chloride and domiphene bromide.

**Claims**

1. A physiologically acceptable, germicide-free, water-based clear mouthwash comprising

(a)    0.1 to 0.5 % weight of an essential oil flavour consisting of spearmint or peppermint or a mixture thereof;

(b) 0.1 to 0.6 % b weight of an alkyl sulphate anionic surfactant mixture wherein said mixture consists essentially of a dodecyl sulphate salt and a tetradecyl sulphate salt wherein the cationic moiety of said salts is sodium, potassium, magnesium, ammonium or substituted ammonium ions, and said dodecyl sulphate is present in said mixture in a weight ratio to said tetradecyl sulphate of 75 : 1 to 1 : 1; and

(c) 5 to 20 % by weight of ehtanol.

2. A mouthwash as claimed in claim 1 wherein the weight ratio of the dodecyl sulphate to the tetradecyl sulphate is 4 : 1 to 1 : 1.

3. A mouthwash as claimed in claim 1 or claim 2, comprising 0.1 to 3 % by weight of a nonionic emulsifiers.

4. A mouthwash as claimed in any of the preceding claims, comprising 5 to 25 % by weight of a humectant.

5. A mouthwash as claimed in any of the preceding claims, comprising 10 to 15 % by weight of ethanol.

6. A mouthwash as claimed in any of the preceding claims, comprising 45 to 85 % by weight of water.


## Patentansprüche

1. Ein physiologisch annehmbares, germizidfreies, klares Mundwaschmittel auf Wasserbasis enthaltend

(a) 0,1 bis 0,5 Gew.-% eines Geschmackstoffes auf der Basis eines ätherischen Öls bestehend aus Minze oder Pfefferminz oder einer Mischung davon;

(b) 0,1 bis 0,6 Gew.-% einer anionischen Alkylsulfat oberflächenaktiven Mischung bestehend im wesentlichen aus einem Dodecylsulfatsalz und einem Tetradecylsulfatsalz, wobei der kationische Bestandteil dieser Salze Natrium-, Kalium-, Magnesium-, Ammonium- oder substituierte Ammoniumionen sind und das Dodecylsulfat in der Mischung anwesend ist in einem Gewichtsverhältnis zu dem Tetradecylsulfat von 75 : 1 bis 1 : 1 und

(c) 5 bis 20 Gew.-% Ethanol.

2. Mundwaschmittel gemäß Anspruch 1, worin das Gewichtsverhältnis von Dodecylsulfat zu Tetradecylsulfat 4 : 1 bis 1 : 1 beträgt.

3. Mundwaschmittel gemäß Anspruch 1 oder 2 enthaltend 0,1 bis 3 Gew.-% eines nichtionischen Emulgators.

4. Mundwaschmittel gemäß einem der vorgehenden Ansprüche enthaltend 5 bis 25 Gew.-% eines Feuchthaltemittels.

5. Mundwaschmittel gemäß einem der vorgehenden Ansprüche enthaltend 10 bis 15 Gew.-% Ethanol.

6. Mundwaschmittel gemäß einem der vorgehenden Ansprüche enthaltend 45 bis 85 Gew.-% Wasser.


## Revendications

1. Un collutoire clair à base d'eau physiologiquement acceptable, exempt de germicide, comprenant:

(a) de 0,1 à 0,5 % en poids d'un parfum d'huile essentielle constitué de menthe verte ou de menthe poivrée ou de leurs mélanges;

(b) de 0,1 à 0,6 % en poids d'un mélange d'égents tensio-actifs anioniques alcoyl sulfates, ce mélange étant constitué essentiellement d'un sel d'acide dodécyl sulfurique et d'un sel d'acide tétradécyl sulfurique, la portion cationique de ces sels étant constituée d'ions de sodium, de potassium, de magnésium, d'ammonium ou d'ammonium substitué, et le dodécyl sulfate étant présent dans le mélange dans un rapport en poids avec le tétradécyl sulfate compris entre 75 : 1 et 1 : 1; et

(c) de 5 à 20 % en poids d'éthanol.

2. Un collutoire selon la revendication 1, dans lequel le rapport en poids du dodécyl sulfate au tétradécyl sulfate est compris entre 4 : 1 et 1 : 1.

3. Un collutoire selon la revendication 1 ou la revendication 2, comprenant de 0,1 à 3 % en poids d'un émulsionnant non-ionique.

4. Un collutoire selon l'une quelconque des revendications précédentes, comprenant de 5 à 25 % en poids d'un humectant.

5. Un collutoire selon l'une quelconque des revendications précédentes, comprenant de 10 à 15 % en poids d'éthanol.

6. Un collutoire selon l'une quelconque des revendications précédentes, comprenant de 45 à 85 % en poids d'eau.